# EUROPEAN PATENT SPECIFICATION

(11) **EP 3 218 836 B1**
(45) Date of publication and mention of the grant of the patent: **04.09.2024**
(21) Application number: 15859120.6
(22) Date of filing: 11.11.2015
(51) Int. Cl.: G16H 10/20, G16H 50/30, G16H 50/20

(54) **A METHOD AND A PROCESSOR FOR DETERMINING HEALTH OF AN INDIVIDUAL**
VERFAHREN UND PROZESSOR ZUR BESTIMMUNG DER GESUNDHEIT EINER PERSON
PROCÉDÉ ET PROCESSEUR PERMETTANT DE DÉTERMINER LA SANTÉ D'UN INDIVIDU

(30) Priority: 11.11.2014 AU 2014904523
(43) Date of publication of application: 20.09.2017
(73) Proprietor: Well Universal Pty Ltd, Sydney, New South Wales 2000 (AU)
(72) Inventor: WILD, Travis Leigh, Sydney, New South Wales 2000 (AU)
(74) Representative: Patel, Binesh
(86) International application number: PCT/AU2015/050701
(87) International publication number: WO 2016/074035

(56) References cited:
- WO-A1-2014/071145
- US-A1- 2002 035 486
- US-A1- 2013 041 290
- US-A1- 2013 262 357
- US-A1- 2013 262 357
- US-A1- 2014 206 949
- US-A1- 2014 257 852
- US-B1- 8 880 174

## Description

### Technical field

The disclosure herein generally relates to a method and a processor for determining the health of an individual.

### Background

Document US2014257852A1 discloses receiving user input selections from a plurality of inquiries generated to receive user input, generating at least one numerical score corresponding to each user input selection, incorporating the at least one numerical score with a user's primary health score; determining whether the user's primary health score is at least one of above or below at least one predetermined health threshold, and creating a notification alert invoking a health care action if the user's primary health score exceeds or undercuts the at least one predetermined health threshold.

Current health screening tests may have low sensitivity. Health data currently being collected is not that with the most sensitivity possible. Current health screening tests may have insufficient range to be the most useful screening tool. They may have a limited range of inputs and, as a result, a limited range of outputs.

The current available data that may be possible to be collected on any one individual may be too much for any physician or practitioner to track for more than a very limited number of patients. There is currently no unified screening test and system that can collate all the available data on an individual in an automated fashion, extract the relevant detail and present it to the individual and practitioner for evaluation and treatment recommendations.

In addition, we currently may have poor methods to sensitively detect wellbeing and illness in large numbers of people and over large areas in `real-time'. This may mean that we are not tracking illness and the transfer of disease as we could, we may have very limited utility in researching the causes of disease on a population scale, and our public health measures may be significantly delayed.

The lack of the above, especially in regards to sensitivity, may result in delayed diagnosis and intervention, and therefore significantly worse outcomes and greater health care expense.

Our current health and medical system is `symptom dependant', and as a result we are operating in a way that is too late for the earliest possible intervention with disease and the associated health outcomes are much worse than they could be.

A current health and medical paradigm typically comprises the steps:
1. Person feels unwell.
2. Person presents to practitioner.
3. Practitioner determines `most likely diagnosis' and a list of differential diagnoses.
4. Practitioner orders confirmatory tests.
5. Confirmatory tests discover indicators 'outside normal range' of possible healthy human levels.
6. Treatment commenced.

Sometimes steps 4 & 5 are eliminated, but the cycle in the paradigm begins at step 1: The person feels unwell. By this stage, it may already be too late. By the time a person feels unwell and has noticeable symptoms, a disease may already be significantly advanced. The person's normal homeostatic regulatory functions and/or their immune system may have already begun to fail and the person may have gone beyond their adaptive capacity. They are already unwell.

Current biomedical, pathological and physiological tests often use the concept of the *'normal human range'.* Only if a test result is outside of normal human range is it conventionally considered that there is a problem.

Most of medicine and physiology is geared towards the detection or confirmation of a specific disease or condition. It generally involves one measurement, or a small number of measures, to confirm a singular disease state and treatment protocol: a small number of inputs to confirm a singular or small number of 'outputs'.

It may not be practical for a practitioner to manually track all of the possible measurable health parameters for an individual which could involve reading dozens of different reports, and collating and mapping the individual readings. This may result in long appointment times. There may not be enough practitioners to manually process large amounts of data for large numbers of people.

That is to say, we currently have testing that could save innumerable lives, dramatically improve health outcomes and lead to greatly reduced health costs for governments and individuals, but we don't have the numbers of practitioners that could hope to manually sift through and collate the relevant data to make these better outcomes possible. The individual testing costs remain quite high and are out of the range of most incomes.

There is currently no worldwide real-time measure of health and wellness for populations, other than hospital bed occupancy (measuring sickness and/or accidents rather than wellbeing).

Large-scale studies are being performed and data is being tracked, but it is at the mercy of the focus of the investigator seeking to measure a specific range of health variables, rather than simply measuring a very large number of parameters and `seeing what the data tells you'. When such large-scale studies are performed, the results are most frequently published months or years after the fact. This gives valuable data about the causes of illness, but nothing that can be acted upon in the moment.

Without exhaustive real-time data being collected and collated relative to geographic location, age, and so on, we are missing extraordinarily valuable data on topics such as:
- How wellbeing is being influenced by external factors: temperature, seasons, environmental factors and so on.
- The transmission of infectious disease per location and demographic variable.
- How things like published news, government announcements, and so on are influencing wellbeing.
- Specific, detailed and 'equal' comparisons of the wellbeing of people between different locations and under different external influences.

As a result, public health policy is always `lagging significantly behind' actual events as they are occurring and influencing the population - dramatically reducing its possible effectiveness and utility.

### Summary

Disclosed herein is a method for determining health of an individual.

In the context of this specification, health of the individual encompasses physical health of the individual, mental health of the individual and emotional health of the individual.

In an embodiment of the invention, there is provided a processor for determining health of an individual as claimed in claim 1. Some optional features of the invention are as claimed in the dependent claims.

Any of the various features of each of the above disclosures, and of the various features of the embodiments described below, can be combined as suitable and desired.

### Brief description of the figures

Embodiments will now be described by way of example only with reference to the accompanying figures in which:
Figure 1 shows a block diagram of an architecture of an embodiment of a processor for determining health of an individual.
Figure 2 shows the processor connected via the communications interface 22 to a computer network.

### Description of embodiments

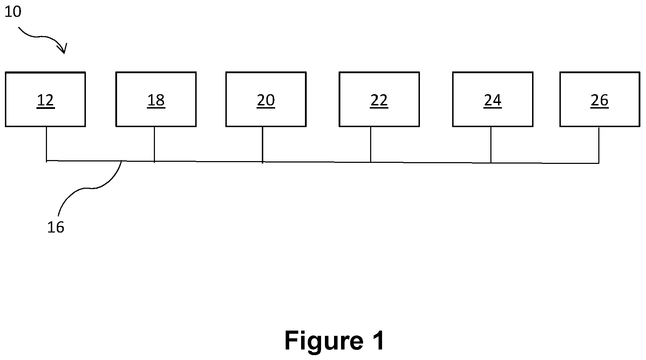

Figure 1 shows a block diagram of an architecture of an embodiment of a processor for determining health of an individual, the processor being generally indicated by the numeral 10. The processor 10 includes a suitable microprocessor 12 such as, or similar to, the INTEL XEON or AMD OPTERON microprocessor connected over a bus 16 to memory which includes a suitable form of random access memory 18 of around 1GB, or generally any suitable alternative capacity, and a non-volatile memory 20 such as a hard disk drive or solid state non-volatile memory (e.g. NAND-based FLASH memory) having a capacity of around 500 Gb, or any alternative suitable capacity. Alternative logic devices may be used in place of the microprocessor 12. Examples of suitable alternative logic devices include application-specific integrated circuits, field programmable gate arrays (FPGAs), and digital signal processing units. Some of these embodiments may be entirely hardware based. The processor 10 has at least one communications interface. In this embodiment, the at least one communications interface 22 comprises a network interface in the form of an Ethernet card, however generally any suitable network interface may be used, for example a Wi-Fi module. The network interface 22 is configured, in this but not necessarily all embodiments, to send and receive information in the form of data packets. The data packets are in the form of Ethernet frames that have an Internet Protocol (IP) packet payload. The IP packets generally have a Transmission Control Protocol (TCP) segment payload, although any suitable protocol may be used. In the present embodiment, the TCP segments may carry hypertext transfer protocol (HTTP) data, for example web page information in HTTP, for example, or a HTTP request or a HTTP response. The HTTP data may be sent to a remote machine. In alternative embodiments, however, proprietary protocols and applications may be used, or generally any suitable protocol (for example SONET, Fibre Channel) or application as appropriate.

The processor may include one or more input/output interfaces 24 in the form of, for example, a universal serial bus (USB), thunderbolt interface, PCIe, or generally any suitable input/output interface. In this but not all embodiments the processor 10 supports a human machine interface 26 e.g. mouse, keyboard, display etc. The data packets may be alternatively or additionally sent and/or received by the input/output interfaces 26, for example a PCIe or USB interface.

The processor alternatively may be a virtual processor, a cloud based service, or a distributed service, for example. Generally the processor may take any suitable form.

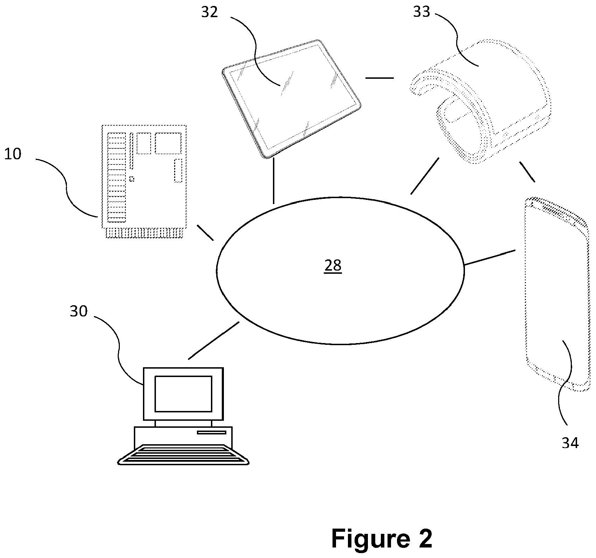

Figure 2 shows the processor 10 connected via the communications interface 22 to a communications channel, computer network 28 in the form of the Internet. A plurality of information collection devices, for example computational devices 30, 32, 33, 34 are in communication with the processor 10 via the network 28. The plurality of information collection devices 30, 32, 33, 34 may be configured to send information about the individual to the processor. The plurality of information collection devices may be configured to generate information about the individual to the processor. The information may be generated by a measurement of the individual, for example, by a physical test, which may be performed by the information collection devices or provided to the information collection devices from another device. The information is about characteristics of the individual, for example blood pressure, happiness score, etc. The computational devices 30, 32, 33 and 34 may take any suitable form, examples of which include a personal computer 30, a smart phone 34 for the individual, a tablet computer 32 for the individual, and a smart watch 33 for the individual. The computational devices 30, 32, 33, 34 may cooperate with the processor 10 to execute a questionnaire, for example a questionnaire for the individual via a web browser on the computational device. The computational devices, for example the smart phone 34, may have at least one of a GPS receiver or other location service, an accelerometer, and sensors that may measure, for example, blood pressure, electrocardiography (ECG), heart rate, blood oxygen, temperature, and lung function. The smart watch 33 may be configured to measure an aspect of the individual, and may or may not be in wireless communication with the smart phone 34 or tablet computer 32 (via a Bluetooth connection, for example). The smart watch 33 may communicate information about the individual to the processor 10, in some embodiments via the smart phone and/or table computer. Alternative information collection devices include an Internet connected global positional system device for location on the individual, an Internet connected personal heart rate and/or blood pressure monitoring device and other personal health indicator monitoring devices. The information collection devices may comprise at least one subcutaneous monitor in the form of, for example, at least one subcutaneous physiological monitor. The subcutaneous monitor may be in wireless communication with the smart phone, for example, which receives information from the subcutaneous monitor and then sends the information to the processor 10. The information collection devices may include a data server 30, for example a pathology laboratory data server, which may be configured to send to the processor 10 information about the individual. The information may comprise, for example, medical test results, questionnaire test results, or generally any information about the individual that may be used in the assessment of the individual's health. The test results may be manually entered into the pathology laboratory server 30 or electronically sent to the server 30 by test apparatus that performs a test on a sample of the individual, for example.

The processor 10 is configured to execute the steps of an embodiment of a method for determining health of an individual. The method may be coded in a program for instructing the processor 10. The program is, in this embodiment stored in the non-volatile memory 20, but could be stored in FLASH, EPROM or any other form of tangible media within or external of the processor. The program generally, but not necessarily, comprises a plurality of software modules that cooperate when installed on the processor so that the steps of an embodiment of the method are performed. The software modules, at least in part, correspond to the steps of the method or components of the processor 10 described above. The functions or components may be compartmentalised into modules or may be fragmented across several software modules. The software modules may be formed using any suitable language, examples of which include C++ and assembly. The program may take the form of an application program interface or any other suitable software structure.

For example, the processor may have a change information module configured to determine if change information indicative of a change in a plurality of characteristics associated with the individual satisfies a predetermined change condition. The predetermined change condition may be, for example, that a health index has changed by more than a predetermined value, which may indicate that the individual is suffering from a disease. The predetermined condition may be indicative of the commencement of the disease before symptoms of the disease occur.

Alternatively, it may be that one health-index (for example a cardiovascular index) has increased, and another health-index (for example a mental stress index) has decreased. The health index may be a composite of health indexes ("composite health index"), for example a cardiovascular index and a stress index. The processor may have a health index generator configured to receive a plurality of values for a plurality of characteristics associated with the individual and generate the health index from the plurality of values by, for example, taking an average of the plurality of values. Some of the values may be adjusted (weighted) before the average is taken. The health index generator may be configured to generate a plurality of indexes.

The collection of characteristics associated with the individual will now be described further.

The 'output' of the method in the form of individual health information may be sent by the processor 10 to a computational device, for example a smart device, tablet or computer, and the health information displayed on an electronic display thereof. The image may be generated by an application or 'App' running on the computational device. Extensive and up-to-the-moment health data may be presented on the smart device. Appropriate action may be taken by the individual or their health professional.

A multi-factor self-report questionnaire (and sub-questionnaires if indicated) may be completed either online via the Internet and computer interface, or using an `App.' and tablet computer or smartphone, for example.

Biomarker physiological inputs may be captured with a device having one or more features common to, for example, a heart rate monitor, a GPS unit or other personal monitoring devices.

An information collection device may be in the form of small device, carried in either a pocket or carrier device such as an armband, or to be incorporated into smartphones or a smart watch. Sensory devices may attach around the chest such as the on-body strap of a heart rate monitor, enabling the detection of heart rate and respiratory rate, amongst others. This device may contain an accelerometer and gyroscope as well as GPS capability to determine movement: gross as well as fine motor balance and posture. This device may also enable a person's sleep to be monitored (movement as an indicator of sleep stage).

An information collection device may comprise a 'cuff' around the upper arm that may inflate and deflate periodically to determine blood pressure. The information collection device may comprise a finger-top 'hood' or smart watch may detect oxygen saturation levels. Skin temperature and conductivity may be detected, and so on, for example by a `finger print sensor' in a smart watch or home entry system, detecting biomarkers every time they activate their phone, make a purchase or enter their home.

The above information may be wirelessly delivered to the device, which can give instantaneous feedback to the user about the parameters measured and will in turn inform our central servers.

Further details generated by bodily function tests may be received: blood, saliva, urine, EEG, EMG, thermography and so on can intermittently be delivered to the processor 10 via a computer interface. Interfaces may be provided for screening operators or laboratory providers to send data about the subject directly into the processor 10, for example an API. Examples of different measurements which may be used to provide physiological information include heart rate measurements, heart rate variability measurements, respiratory rate measurements, respiratory rate variability measurements, blood pressure measurements, physical movement observations, cortisol level measurements (measured in blood or saliva), skin conductivity measurements, skin temperature measurements, skin or hair analysis, DNA analysis, blood oxygen saturation measurements, surface electromyography (surface EMG) measurements, electroencephalography (EEG) measurements and measurements other physiological indicators of stress able to be determined by analysis of a person's blood, saliva or urine. The saliva, blood, urine, skin, hair and DNA measurements can be carried out through conventional laboratory testing or via nanotechnology, where for example, nanotechnology sensors can be used for single-blood drop measures, can be incorporated in a transdermal patch, can be injected subcutaneously or circulate within the body of the individual or may incorporate the use of a subcutaneously embedded microchip or wire-enabled sensor.

Furthermore, 'smart clothing' can also be utilised, which can include pants/trousers, underwear, socks, shoes, shirts/T-shirts, gloves, hats/caps/helmets, glasses, watches, smart-watches, wrist and ankle bands, as well as adhesive patches. The 'smart clothing' is embedded with various sensors, including electrical signal, conductivity (galvanic conductance and resistance), accelerometers, force, temperature, chemical sensors and nanotechnology sensors can be used to provide physiological information.

Embodiments may comprise subcutaneous physiological monitors that can provide some additional data, wherein in use a very thin wire inserted through the skin or a microchip, for example. These devices may offer greater numbers of physiological, biomarker and protein detection inputs and may be used instead of the many biometric input measures mentioned herein.

The individual's car and 'smart house' systems may send to the processor 10 information regarding driving (acceleration, braking, steering inputs, and so on), movement around the home, television watching characteristics, water consumption, time of going to bed (versus sleep time gives an indication of the time taken to get to sleep and a person's natural circadian rhythm). The individual's computer may collect data about eye movement, facial expression, facial blood flow, and so on. If the individual wishes an even greater level of transparency and clarity, their Internet search history may be monitored as well as their purchasing/shopping data (specific data held on their own secure device) that may be able to collate `data patterns' with usual health profiles and parameters. This may be able to inform a person 'the last time you purchased x, you were very stressed; please complete our stress assessment' or, `it seems as if your Internet search pattern is slower today than it has been; are you tired?'.

Users (individuals) may be able to inform exercise equipment (their own or within professional gymnasium) connected to the Internet to update their own Wellbeing Profile. This may give information regarding a subject's expenditure (and therefore energy levels) on any device, as well as other fitness parameters (power output including left-right balance, calorie usage, and so on), in addition to the types of equipment chosen and time in between equipment (relates to energy levels and behaviours).

Genetic and epigenetic assessment information may also provide external input specialist test data and will be incorporated into the processor 10 in a similar fashion to the regular blood tests.

Practitioners with access may input or update the user's health history and consultation/diagnosis/treatment notes. The individual may also wish to record their consultations with their smartphone and the summarized file notes will be transcribed into their stored continuous health history.

The various information streams may then be combined by the processor, for example, to calculate a composite Health or Wellness Index quotient. In another embodiment, the various information streams are combined on the smart device (e.g. tablet or smart phone).

Specific sub-components may also be grouped together to form the specific sub-Indexes or component indexes and parameters such as biological age measure, and so on. Analysis and recommendations may then be delivered via the Internet to the individual's devices, their guardian, approved health care provider or insurer for example.

The output from the psychometric self-report questionnaire may improve sensitivity. Physiological and cognitive/ performance measurements may be monitored according to their own sub-indexes and may be mapped onto the questionnaire components where indicated. Behavioural data may be tracked and statistically correlated to other 'deflections' detected by the processor 10 in other parameters.

The psychometric questionnaires, cognitive function tests, biomarkers and behavioural inputs may act as initial screening that may be more broad than existing screening:
- The general psychometric questionnaire may detect a person's self-report level of wellbeing and health-related lifestyle. It may also house general screening questions about mental illness, depression, anxiety, stress levels, and so on. If these general screening questions are 'triggered', the subject may be instantly requested to complete the indicated additional self-report questions such as a specific anxiety and depression questionnaire.
- First-round biomarker tests may detect whether the measured parameters for the individual are `within the general human normal range'.
- Cognitive function tests may initially detect major impairment and then, if indicated, suggest additional tests.
- Behavioural data may show movement and social interaction data, enabling the detection of severe or extreme behavioural cues.
- Genetic and epigenetic testing may suggest additional screening tests and protocols that should be applied.

Specific components of the above may be collated into a singular `wellness or health score or index', as well as producing multiple wellness profiles and scores such as an `Emotional Health Index', a `Mental or Cognitive Health Index', `Biological Age', and so on.

Over the next weeks and months (or other relevant time frame, depending upon the applied testing protocols and frequency) the processor 10 may collate and map the subject's personal normal ranges in all categories (psychometric, biomarker, cognitive, behavioural) that may enable the ongoing detection of the subject beyond their own 'normal' in addition to the population 'normal' values. The processor 10 may `learn that person's normal'. Having established the personal normal values, the processor 10 may able to apply the composite index algorithms to determine the `group fluctuations' within this person: a much greater sensitivity may be established.

In addition, the processor 10 may determine the fluctuations in each of the measured parameters and correlate them with the person's idiosyncratic or 'personal behaviour map'. For example, the processor 10 may detect that with a certain combination of parameters in a person (biomarker, psychometric, cognitive or all three) that they tend to move about their house, drive their car, exercise, type, speak, use certain 'apps' in certain ways, scan the internet or flick between television shows in a certain way, or that they purchase certain combinations of items, travel to certain places, interact with certain individuals, sleep and wake at different timings, or display certain sleep cycle characteristics that they carry a certain type of facial expression, and so on. As the processor 10 may learn these personal behavioural cues of an individual user relative to other health indicators over months and years, these behaviours themselves (individually or in concert) may become the indicators for the subject's wellbeing. The longer the person is measured, in every category, the more data about their own 'normal' and behaviours may be collected, and the more sensitive the processor 10 becomes to them.

Within a few months of commencing to measure an individual, the processor 10 may have defined the 'normal range' of the person's major health variables and through combining the inputs as a composite index may be able to detect movement towards ill health *within* the person's personal normal range. The processor 10's sensitivity may be to the degree that it can detect the individual beginning to adapt to a virus or that they may be becoming unwell in some way. It may then inform the person that their indicators are suggesting that they are `a little off, or possibly becoming unwell' and may offer suggestions as to lifestyle, nutrients or practitioners that could be considered. The subject can then request further detail and the specifics will be shown to them. The processor 10 may be able to suggest that `last time you showed these parameters you attended Dr X (or you got to bed much earlier than usual, etc.) and they returned to normal range within 2 days'.

An embodiment combines a multi-factor psychometric questionnaire about perceived health and wellbeing indicators with at least one of biological, physiological measures, physical measures, cognitive function and performance tests, behavioural input data from multiple sources, environmental inputs and social and economic indicators to determine an individual's health or wellness profile, score or index. The input data from each individual may be separated into distinct units or subgroups that articulate precisely the way in which the subject is most unwell/well. The scores from participating individuals are collated continuously into a worldwide database to produce a worldwide wellness index that can be separated into distinct geographic regions or via other defining characteristics or demographic variables such as gender, income, educational attainment, and so on.

The psychometric or self-report questionnaire asks an individual about their subjective experience of health and wellness-related signs, symptoms or indicators across a spectrum of wellbeing indicators: physical, mental/cognitive, emotional, and so on. It also elicits detailed lifestyle and health behaviour information regarding nutrition, exercise, health habits, work, desire for change, a person's family health history and so on. These self-report parameters may span the full range of the measureable aspects of wellbeing and health behaviour. Depending upon the answers given in the first general screening questionnaire, when indicated the user may be presented with subsequent 'specific interest topic' questionnaires: these additional questions will be triggered automatically by the processor 10. For example, if the general screening questionnaire detects that a person may be showing some signs of depression or anxiety, a specific 'depression and anxiety' psychometric questionnaire will be presented in order to fully elucidate their depression and anxiety parameters.

These specific interest questionnaires may be available to other 'non-indicated' users as well (those very interested in self-measurement). The design of general screening and specific-when-indicated questionnaires adds greatly to the utility of this invention in being able to define the greatest useable detail for the least amount of inconvenience and time on the part of the user.

The additional questionnaires that may be presented may cover aspects including, but not limited to:
- Anxiety, depression, mental illness and so on (specific conditions or states).
- Risk of self-harm.
- Health history (personal), family health history in additional detail. For example, if a person indicates a history of significant illness, additional questions will seek to define the related health history.
- Specific questionnaires related to Anger, Exhaustion/Burnout, Happiness, Social connectedness, Workplace Satisfaction, Stress and so on.
- Health awareness, Health Literacy, Knowledge of behavioural change techniques relating to health, Literacy and Numeracy. (These questionnaires will add efficacy to the delivery of advice - presenting information in the most effective manner for the particular user.)

The biological, physiological or physical measures of wellbeing (biomarkers) may include, but are not limited to, one or more of the following:
- Heart rate, heart rate variability, respiratory indicators such as respiratory rate and variability, blood pressure, skin conductivity, temperature, blood oxygen saturation levels, weight, body mass, metabolic indicators, skin colour detection, and so on.
- Physical movement sensor, gyroscopic and GPS data, step counters, arm and leg movement sensors, bicycle meters, power meters, wrist sensors, sensors in shoes and other clothing, data from exercise or gym equipment, data indicating body balance and movement.
- Surface electromyography (surface EMG), electroencephalography (EEG), electrocardiography (ECG), sleep cycle and timing measurement, indicators of `vagal tone' and HPA axis stimulation.
- Full blood, urine, saliva, faeces, hair and skin analysis. Possible inputs from more than 200 known traceable indicators, chemicals, compounds, proteins, and so on.
- Genetic and familial health information including full genetic and epigenetic assessment data, as well as information regarding relatives and socially-connected others (adopted relatives, etc.).
- Under-skin detection systems, such as those provided by wire through the skin or via microchip.
- Data from whole-body or specific screenings such as MRI, Ultrasound, Bone density tests, pathology tests, health histories with professional providers, diagnoses and reports, and so on.

The index may have the capacity to include the above. Individual inputs may be weighted and scaled onto the Wellness Index according to their relevance to whole-health and wellness.

Data from these inputs may act as a `health watch' processor 10, with initial monitoring detecting those indices that are `within normal range' and alerting the individual to movements approaching or `beyond normal human range'. Subjects may be encouraged to have these parameters measured regularly. As the processor 10 receives ongoing data input from a subject, it may be able to detect a person's own specific 'individual normal range' of the various inputs: this then enables detection beyond the subject's `individual normal range' (far more sensitive than whole-population 'normal values'). When singular inputs are then combined into a multiple-input composite index for the subject's wellbeing, it allows for detection of movement towards dysfunction *within their own personal or individual normal range:* this offers the ability to detect an individual *as they are becoming unwell* (a function available with this invention for the first time in history) and much earlier than any other screening device available.

The indices that may be more relevant to a person's detectable level of whole-health and wellness, such as heart rate variability, sleep cycle, stress indicators, oxygen saturation, respiratory rate and depth and so on may be scaled onto the person's wellness index.

Data from all health assessments and reports may be stored to act as a unified repository of information about that individual that all health providers worldwide may be able to access, given permission from the subject.

Cognitive function or performance tests may comprise online tasks, but may alternatively or additionally comprise interaction with 'smart watches', phones or other devices. They may involve tasks such as memory tests, reaction-time testing, decision making, learning capacity, assessments for 'optimism' or other aptitudes (inherent or learnable), and so on. They may also include cognitive assessments such as eye-movement tracking and the measurement of types of data that the subject preferentially attends to: for example, as a subject browses an online 'news' provider, the eye movement tracking device may be able to determine the types of content that the subject is concentrating upon and for how long, which may provide data relevant to cognitive function, mental or emotional state or ability to hold attention on a singular subject.

Behavioural data may include information regarding an individual from inputs received through, but not limited to:
- Phone, tablet, computer and other devices via Bluetooth, NFC or other communication channels.
- Movement of the subject through GPS or other modes of `location detection'.
- `Smart home' detection devices, car driver and passenger monitoring, driving style detection, workplace monitoring, television or computer individual recognition (eye movement, facial expression, posture), gym equipment detecting how a person moves around a gym or between equipment, detection of individuals through facial recognition and other movement tracing devices, posture and `mood detection' through body and facial recognition devices, and so on.
- Internet search history, use of specific Applications or combinations of them, purchasing and shopping data, travel history, and so on.

The ability to detect how a person moves within and outside their home, their proximity to other individuals (through the detection of other devices) and their repetition in doing so, their internet and Application use, the reading of a subject's mood via their computer sensing their posture and facial expression, and their purchasing data may provide health-related data for the detection and measurement of wellbeing. This information may provide early detection of illness, emotional state and `energy levels' for individuals, as well as being able to trace the source of communicable diseases.

Environmental inputs may include, but are not limited to, the temperature of the immediate environment of the subject as well as outside temperature, measurement of sunshine and UV, time spent outdoors, wind, humidity, barometric pressure, inputs for traffic, public unrest, combined external stress levels.

Economic indicators may include, but are not limited to inputs related to Gross Domestic Product of the region or country in which the subject resides, Employment Figures, ratings for Disposable Income, Cost of Living, Cost of Housing, Consumer Confidence, share or stock market Indices and other economic measurements or indicators.

The combined input from the self-report parameters of the psychometric questionnaire(s), the physiological or biometric measurements, the cognitive/ performance indicators, and the behavioural data are then logged onto a combined health and wellness index able to give the subject an indication of wellbeing, more accurate than any single indicator alone or combination of indicators currently in existence. The additional data regarding economic and environmental data, combined with the personal data, provides for an unprecedented level of ability to research the health and wellbeing characteristics of populations.

The measurements may be taken continuously or at intervals across a reference period, depending upon the test or measurement in question.

The combined data for a particular subject may then be subdivided or separated into specific sub-groups, such as but not limited to:
- A whole health or wellness measure, rating, scale or Index.
- A measurement of Biological Age.
- Longevity forecast.
- Morbidity forecast (disease likelihood).
- Physical and Physiological Wellbeing Index.
- Mental or cognitive Wellbeing Index.
- Emotional Wellbeing Index, that in itself can be separated into separate indexes relating to Anxiety, Depression, Anger, Exhaustion or Burnout, Happiness, Creativity, and so on.
- Social Wellbeing Index.
- Behavioural Health Index, incorporating health habits, exercise, nutrition, health literacy, knowledge of how to improve health behaviour and so on.
- Viral infection.
- Bacterial infection.
- Asthma.
- Inflammation.
- Sudden Infant Death Syndrome (SIDS).
- Heart disease.
- Physical impact.
- Anaphylaxis
- Ovulation/Menstruation/Labour.
- Various malignancies.
- Migraine.
- Diabetes/diabetic shock.
- Attention Deficit Hyperactivity Disorder (ADHD).
- Distress.
- Consciousness.

With this specific and detailed knowledge, embodiments may monitor an individual's wellbeing more closely than has ever been possible before, 'learning' each person's individual and precise 'normal range', and able to detect movement towards illness *within an individual's normal range,* provides for an exceptional level of sensitivity as an early warning detection device for health indicators in every domain.

Each user's data may be logged onto a unified database that may enable the processor 10 to calculate average health and wellness levels and associated normative data and to monitor fluctuations occurring in `real time' in populations according to geographic location, occupation, gender, age and many other variables and influences. This may allow for an enormous number of research possibilities as to the influences upon health and wellbeing, and should as an index offer great insight for organizations such as governments (local, regional and federal), `liveability' measurement systems, town planners and developers, health and life insurance companies, and so forth.

The *'normal human range'* of C-Reactive Protein (CRP), for example, has a mean (average) value of 0.8mg/L in normal healthy adults. But this average 'normal' varies between actual individual healthy people to quite a large degree (some people's average or 'normal' CRP is 0.3mg/L, and other healthy people's 'normal' may be 1.3mg/L). CRP values also vary slightly on any particular day within the one healthy person, and with different tests depending upon the idiosyncrasies of sampling.

In medicine, *normal human range* has been necessary for infrequent or one-off blood tests. If someone presents to you as a physician as 'ill' and you now want to test their levels, you have no idea where their specific 'normal' level is (you don't have that data available) so the tests have to account for the whole possible `range of normal' results amongst all healthy humans. The applied tests may not stand out as 'abnormal' unless you have gone well beyond the furthest reaches of the possible human range of healthy, and only then can the alarm bells start to ring.

The same is generally true for every biomarker: every person's actual 'normal' is different to everybody else, but the one-off traditional blood or biomarker tests have to account for the whole range of human 'normal' values. These *normal human range* values, by the mere fact that they need to account for every possibility while we are all actually different, are by their nature insensitive. Very few people, in fact, score 'average'.

An embodiment has continuous monitoring, using continuous monitoring devices such as, for example, heart rate monitor, skin temperature monitor, and conductivity monitor, accelerometer gyroscope monitor, and subcutaneous monitor. Monitoring a person continuously, or a lot, gives the ability to define their actual *individual normal range.* If we use the example of heart rate, the Mayo clinic publishes that the healthy human resting heart rate varies between 60bpm to 100bpm. This is a massive range of 'normal'. If we track an athlete we might find that their specific individual average resting heart rate is 65bpm, and they have a `personal range' of resting heart rate whilst healthy of 63bpm to 70bpm. We don't need to wait until that person's heart rate has exceeded 100bpm to know that 'something is up' with them: once it has exceeded 71bpm we can have alarm bells being triggered.

Using embodiments that have continuous monitoring, we no longer have to wait for a person to move beyond the *normal human range* of the `whole herd' in order to be detected.

Again, the same is true for every biomarker. This advance of continuous monitoring has meant that we can detect the personal or individual 'abnormal' *within* the range that was previously considered to be 'normal' for all humans: an advance in sensitivity and ability for early detection and intervention.

An embodiment uses an index of human data. Using the example of the athlete above with a normal individual 'average' heart rate of 65bpm (range of 63 to 70bpm). If his heart rate goes to 66bpm, to the current continuous monitoring models this means nothing. However, if it happened that his respiration rate also goes up by 1% (again within his 'normal range'), his skin temperature goes up by 0.2%, his eye movements alter by 1%, and so on with 20 of his 150 biomarkers, this tells us that 'something is up' with him. These individual scores are all well within his `personal normal ranges', but combined together they can give us an indication of a person `beginning to depart from their own normal' and give us the earliest possible indication of dysfunction not only before obvious symptoms have developed, but also before illness has even developed. There is no need to wait until he has a heart rate 71bpm to `ring the warning bell' and to suggest intervention, we can do so as soon as it could be possible with a human. This gives the earliest possible opportunity for intervention and therefore the greatest possible health outcomes.

An embodiment uses and may collect behavioural data. The way we move, the way we interact, the types of websites that we visit, our eye movement characteristics, the number of calls of texts that we make, the frequency of key words in our text messages or Internet searches, the way we accelerate and brake while we drive, and so on, may contain information about a subject's internal state. When we are anxious, sad, depressed, feeling poorly, we may move certain ways, buy certain things and go certain places. When we're agitated we may drive more aggressively and some of us may gesticulate more vigorously. When we change our mood or feelings, or even begin to become ill, our movement and behaviours may change and it can also begin to show in our facial expressions and posture. (Walking just a little more slowly than normal might be the first expression of a viral infection.).

Technologies including, for example, smart phones, tablets, watches, and so on, can detect movement and speed of movement (GPS, accelerometer, gyroscope, etc.), interaction (proximity of other Bluetooth and NFC units, and the frequency with which they are encountered), can detect facial expressions, posture and blood flow. Smart-houses that have monitors that can detect the way we move around the house and so on, could be a significant source of wellbeing data. A person's shopping lists and Internet search history may give people great insight to their internal state.

Embodiments may also receive information on cognitive function assessed by specific tests and challenges through 'smart devices' (reaction time, memory, and so on).

Mental and emotional state can be assessed through questionnaires.

As a result of being so broad in its number of inputs, an embodiment may be able to detect the wellbeing of individuals in categories that other narrow-range or `specific input devices' might miss. For example, even if one goes as far as measuring of every known biomarker as an indicator of wellbeing and illness, you would still fail to have any sensitivity in the realm of mental or emotional health (beyond the extreme cases), and would also fail to be able to detect behavioural cues to illness that may also be the first signs dysfunction (biological or psychological).

By screening in every category (comprehensive multi-factor questionnaire, very large number of biomarker inputs, cognitive and behavioural), the greatest possible range of detection of health and wellbeing for people may be achieved.

Having a greater range of variables being continuously or frequently monitored (having the greatest number of `data stream inputs') may provide greater sensitivity. Embodiments may be able to determine an individual's *'personal or individual normal range'* and detect the movement beyond their own personal normal, in every category: biomarkers, using the psychometric questionnaires, behavioural data and so on.

An embodiment may collate the individual inputs into a composite index for a person's wellbeing. As described previously, this produces a greater level of sensitivity that is able to detect if several of the person's characteristics are `moving in the same direction' or other predetermined movement pattern at the same time. This may enable the earliest possible detection that a person may be becoming ill, that their adaptive range may be compromised, that they have taken on a virus, or they simply 'may be a little off' in regards to their wellbeing and possibly may be more susceptible to becoming ill, within their 'normal range'.

Having a great number of possible separate and distinct `data streams' into a composite index may result in a measurement device for health and wellbeing that is more sensitive and may be more stable than any other existing device.

Showing a plurality of biomarkers graphically on a large wall screen may give a better `range' than just one characteristic being monitored, but no increase in sensitivity. If one biomarker moves `out of line' of the considered normal range (population or personal) you will see it, but there is no inherent measure of wellbeing or ability to detect movement *within* normal ranges.

Use of a plurality of data in the form of a composite index generally allows detection of useful information about a person's wellbeing within their normal range. It may be difficult to achieve the maximum level of sensitivity, stability and utility through other means.

Generally but not necessarily, the greater number of separate inputs into the composite index, the more sensitive, stable, wide-ranging and useful it will be as a wellbeing indicator.

Sensitivity, stability and additional utility may increase with the number of their separate inputs: more inputs = more sensitivity and stability. The more significant health indicators that are used as inputs the more stable and sensitive the index.

An embodiment may have a very large number of distinct 'biomarker' inputs combined with a multi-factor questionnaire (each included 'factor' acts as a separate input data stream) which may be exhaustive or near exhaustive. This combination means a greater number of distinct inputs, and therefore embodiments may be more sensitive, stable and wide-ranging than existing devices.

In addition to discerning a total Wellbeing or Health Score or Index for a subject, by combining various subgroups of inputs has the ability to define a Health or Wellbeing Profile that separates the various inputs into sub-categories or Indexes that include, but are not limited to:
- A whole health or wellness measure, rating, scale or Index.
- A measurement of Biological Age.
- Longevity forecast.
- Morbidity forecast (disease likelihood).
- Physical and Physiological Wellbeing Index.
- Mental or cognitive Wellbeing Index.
- Emotional Wellbeing Index, that in itself can be separated into separate indexes relating to Anxiety, Depression, Anger, Exhaustion or Burnout, Happiness, Creativity, and so on.
- Social Wellbeing Index.
- Behavioural Health Index, incorporating health habits, exercise, nutrition, health literacy, knowledge of how to improve health behaviour and so on.
- Viral infection.
- Bacterial infection.
- Asthma.
- Inflammation.
- Sudden Infant Death Syndrome (SIDS).
- Heart disease.
- Physical impact.
- Anaphylaxis
- Ovulation/Menstruation/Labour.
- Various malignancies.
- Migraine.
- Diabetes/diabetic shock.
- Attention Deficit Hyperactivity Disorder (ADHD).
- Distress.
- Consciousness.

The 'automated' and combined nature may result in a more sensitive and wide-ranging health monitoring system than is currently available. It may produce an automated `processor 10' that can define health, rather than the requirement for the current symptom-based and driven consultation dynamic. This is turn will mean that medical consultation times can be significantly reduced from those currently in place, at the same time as increasing their precision, consistency and efficacy.

Automation may assist practitioners to incorporate the large amounts of data quickly in a timely fashion. Without automation, practitioners may be unable to cope with the available data and consequently the population may not be able to benefit.

Embodiments may track and report the progress of the subject to the practitioner remotely via, for example, the internet, and in real-time. Alerts may be generated and send by the processor 10 for indications of illness. Data may be collected as to the efficacy of treatment protocols or the uptake of behavioural and lifestyle suggestions. Because all progress may be tracked on a longitudinal basis, the processor 10 may provide benefits for medical research.

Now that embodiments have been described, it will be understood that some embodiments may have some of the following advantages:
- An unclaimed embodiment may provide a unified screening test and system that can collate all the available data related to the health and wellbeing of an individual in an automated fashion, extract the relevant detail and present it to the individual and practitioner for evaluation and treatment recommendations.
- An embodiment may be able to detect ill health on a very wide scale within the adaptive range (or normal range for that individual), maybe before symptoms become apparent and maybe before a person is showing abnormalities within biomarker tests. This may represent an increase in sensitivity.
- An unclaimed embodiment uses health indicators commonly missed, improving performance.
   Many prior art health checks do not account for a huge swathe of health indicators, for example emotional, mental or cognitive state (beyond the most severe chemical imbalance) and completely ignores data from tracking a person's behaviour (beyond his daily exercise monitoring).
- An embodiment may automate combination and/or collation of the available and proliferating data. Other automated unclaimed embodiments that can process and keep track of large amounts of data for large numbers of people may encourage more individuals to take up broad-spectrum testing. Increased participation may increase competition in broad-spectrum analysis and result in a reduction in cost, which may create a larger market. This may result in a more-tested population, creating much better health outcomes and a healthier population.
- Specific data about each individual may be made readily available to any authorized practitioner or researcher, and may offer relatively precise and detailed recommendations regarding health to any user, and the ability to track the effectiveness of any intervention (prescribed or self-administered).
- Embodiments may offer improved range, sensitivity and stability to detect illness as early as possible, providing for earlier intervention, better outcomes, greatly reduced morbidity and mortality rates, reduced cost of health care, less time off work, and greater productivity.
- Unclaimed embodiments may provide data for individuals to not only manage their health better, but to also negotiate greatly reduced and specific health insurance premiums.

It is envisaged that some embodiments of the present invention relates to use in the veterinary field, relevant for both livestock on the commercial scale and domestic animals. In these embodiments, by combining various subgroups of inputs has the ability to define a Health or Wellbeing Profile that separates the various inputs into sub-categories or Indexes that include, but are not limited to:
- Infection.
- Inflammation.
- Distress.
- Anaphylaxis.
- Poison - snake, tick, man-made.
- Ovulation/fertility cycle/labour.
- General health (as per human version).
- Movement - in total.
- Biomechanical balance.

Prior art, if any, described herein is not to be taken as an admission that the prior art forms part of the common general knowledge in any jurisdiction.

In the claims which follow and in the preceding description of the invention, except where the context requires otherwise due to express language or necessary implication, the word "comprise" or variations such as "comprises" or "comprising" is used in an inclusive sense, that is to specify the presence of the stated features but not to preclude the presence or addition of further features in various embodiments of the invention.

## Claims

1. A processor for determining health of an individual, the processor comprising at least one microprocessor and a memory, the memory comprising instructions executable by the at least one microprocessor such that the processor is operable to execute the steps of:
receiving a plurality of values for a plurality of characteristics associated with the individual;
determining the individual's normal range for each of the plurality of characteristics;
combining the plurality of values into a multiple-input composite index;
determining if change information indicative of a change in the composite index satisfies a predetermined change condition;
wherein the predetermined change condition when satisfied is indicative of the movement of the individual's well-being towards dysfunction within their individual normal range;
wherein the normal range of the characteristic information for the individual is learned by the processor by collation and mapping of the characteristic information of the individual over a time frame dependent upon applied respective testing protocols and frequency;
wherein the plurality of characteristics include at least two of heart rate, heart rate variability, a respiratory indicator, respiration rate, respiratory rate variability, blood pressure, skin conductivity, temperature, blood oxygen saturation levels, weight, body mass, metabolic indicators, skin colour, a physical movement characteristic, a brain activity characteristic, a heart activity characteristic, a vagal tone characteristic, and a HPA axis stimulation characteristic, a blood characteristic, a urine characteristic, a faeces characteristic, a hair characteristic, a skin characteristic, a genetic characteristic, an epigenetic characteristic, sleep cycle characteristic, and a bone density characteristic.

2. A processor defined by claim 1 wherein the movement of the individual's well-being towards dysfunction within their normal range is indicative of an abnormal health condition.

3. A processor defined by claim 2 wherein the abnormal health condition comprises a disease.

4. A processor defined by claim 2 wherein the movement of the individual's well-being towards dysfunction within their normal range is indicative of the commencement of the abnormal health condition before symptoms of the abnormal health condition occur.

5. A processor defined by claim 3 wherein the disease indicated is validated by the processor using results from a questionnaire.

6. A processor defined by claim 5 wherein the questionnaire comprises screening questions and further questions determined from the individual's response to the screening questions.

7. A processor defined by claim 1 comprising the step of determining the change information.

8. A processor defined by claim 1 comprising the step of the individual performing an online task.

9. A processor defined by claim 1 comprising the step of monitoring usage patterns of a machine by the individual.

10. A processor defined by claim 1 comprising the step of electronically monitoring the behaviour of the individual.

11. A processor defined by claim 1 wherein the plurality of values are weighted.

12. A processor defined by claim 1 wherein the individual is an animal, including a human being.

## Patentansprüche

1. Prozessor zur Bestimmung der Gesundheit eines Individuums, wobei der Prozessor mindestens einen Mikroprozessor und einen Speicher umfasst, wobei der Speicher Anweisungen umfasst, die von dem mindestens einen Mikroprozessor so ausführbar sind, dass der Prozessor betriebsfähig ist zum Ausführen der folgenden Schritte:
Empfangen einer Vielzahl von Werten für eine Vielzahl von Merkmalen, die dem Individuum zugeordnet sind;
Bestimmen des Normalbereichs des Individuums für jedes der Vielzahl von Merkmalen;
Kombinieren der Vielzahl von Werten zu einem zusammengesetzten Index mit mehreren Eingaben;
Bestimmen, ob Änderungsinformationen, die auf eine Änderung des zusammengesetzten Index hinweisen, eine vorgegebene Änderungsbedingung erfüllen;
wobei die Erfüllung der vorgegebenen Änderungsbedingung auf eine Tendenz zum Abdriften des Wohlbefindens des Individuums in Richtung einer Funktionsstörung innerhalb seines individuellen Normalbereichs hinweist;
wobei der Normalbereich der Merkmalsinformationen für das Individuum vom Prozessor durch Zusammenstellen und Zuordnen der Merkmalsinformationen des Individuums über einen Zeitraum erlernt wird, der von den jeweiligen angewandten Testprotokollen und der -häufigkeit abhängig ist;
wobei die Vielzahl von Merkmalen mindestens zwei von Herzfrequenz, Herzfrequenzvariabilität, einem Atmungsindikator, Atemfrequenz, Atmungsfrequenzvariabilität, Blutdruck, Hautleitfähigkeit, Temperatur, Sauerstoffsättigungsgrad des Blutes, Gewicht, Körpermasse, Stoffwechselindikatoren, Hautfarbe, einem Merkmal der körperlichen Bewegung, einem Merkmal der Gehirnaktivität, einem Merkmal der Herzaktivität, einem Merkmal des Vagustonus und einem Merkmal der Stimulation der HPA-Achse, einem Merkmal des Blutes, einem Merkmal des Urins, einem Merkmal des Stuhls, einem Merkmal des Haares, einem Merkmal der Haut, einem genetischen Merkmal, einem epigenetischen Merkmal, einem Merkmal des Schlafzyklus und einem Merkmal der Knochendichte einschließt.

2. Prozessor nach Anspruch 1, wobei die Tendenz zum Abdriften des Wohlbefindens des Individuums in Richtung einer Funktionsstörung innerhalb seines Normalbereichs auf einen abnormalen Gesundheitszustand hinweist.

3. Prozessor nach Anspruch 2, wobei der abnormale Gesundheitszustand eine Krankheit umfasst.

4. Prozessor nach Anspruch 2, wobei die Tendenz zum Abdriften des Wohlbefindens des Individuums in Richtung einer Funktionsstörung innerhalb seines Normalbereichs auf den Beginn des abnormalen Gesundheitszustands hinweist, bevor Symptome des abnormalen Gesundheitszustands auftreten.

5. Prozessor nach Anspruch 3, wobei die angegebene Krankheit vom Prozessor unter Verwendung von Ergebnissen aus einem Fragebogen validiert wird.

6. Prozessor nach Anspruch 5, wobei der Fragebogen Screening-Fragen und weitere Fragen umfasst, die aus den Antworten des Individuums auf die Screening-Fragen bestimmt werden.

7. Prozessor nach Anspruch 1, umfassend den Schritt des Bestimmens der Änderungsinformationen.

8. Prozessor nach Anspruch 1, umfassend den Schritt, dass das Individuum eine Online-Aufgabe durchführt.

9. Prozessor nach Anspruch 1, umfassend den Schritt des Überwachens der Nutzungsmuster einer Maschine durch das Individuum.

10. Prozessor nach Anspruch 1, umfassend den Schritt der elektronischen Überwachung des Verhaltens des Individuums.

11. Prozessor nach Anspruch 1, wobei die Vielzahl der Werte gewichtet ist.

12. Prozessor nach Anspruch 1, wobei das Individuum ein Tier, einschließlich eines Menschen, ist.

## Revendications

1. Processeur permettant de déterminer la santé d'un individu, le processeur comprenant au moins un microprocesseur et une mémoire, la mémoire comprenant des instructions exécutables par l'au moins un microprocesseur de sorte que le processeur puisse fonctionner pour exécuter les étapes consistant à :
recevoir une pluralité de valeurs d'une pluralité de caractéristiques associées à l'individu ;
déterminer la plage normale de l'individu pour chaque caractéristique de la pluralité de caractéristiques ;
combiner les valeurs de la pluralité de valeurs en un indice composite d'entrées multiples ;
déterminer si des informations de modification indiquant une modification de l'indice composite satisfont une condition de modification prédéterminée ;
dans lequel la condition de modification prédéterminée, lorsqu'elle est satisfaite, indique le déplacement du bien-être de l'individu vers un dysfonctionnement à l'intérieur de la plage normale de l'individu ;
dans lequel la plage normale des informations de caractéristiques de l'individu est apprise par le processeur par un collationnement et un mappage des informations de caractéristiques de l'individu sur une trame temporelle dépendant de protocoles de test respectifs appliqués et d'une fréquence ;
dans lequel la pluralité de caractéristiques comprend au moins deux caractéristiques parmi une fréquence cardiaque, une variabilité de fréquence cardiaque, un indicateur respiratoire, une fréquence respiratoire, une variabilité de fréquence respiratoire, une pression sanguine, une conductivité cutanée, une température, des niveaux de saturation en oxygène sanguin, un poids, une masse corporelle, des indicateurs métaboliques, une couleur de peau, une caractéristique de mouvement physique, une caractéristique d'activité cérébrale, une caractéristique d'activité cardiaque, une caractéristique de tonus vagal, et une caractéristique de stimulation d'axe hypothalamo-hypophyso-surrénalien (HPA), une caractéristique sanguine, une caractéristique urinaire, une caractéristique de selles, une caractéristique pilaire, une caractéristique de peau, une caractéristique génétique, une caractéristique épigénétique, une caractéristique de cycle de sommeil, et une caractéristique de densité osseuse.

2. Processeur selon la revendication 1, dans lequel le déplacement du bien-être de l'individu vers un dysfonctionnement à l'intérieur de sa plage normale indique un état de santé anormal.

3. Processeur selon la revendication 2, dans lequel l'état de santé anormal comprend une maladie.

4. Processeur selon la revendication 2, dans lequel le déplacement du bien-être de l'individu vers un dysfonctionnement à l'intérieur de sa plage normale indique le commencement de l'état de santé anormal avant l'apparition de symptômes de l'état de santé anormal.

5. Processeur selon la revendication 3, dans lequel la maladie indiquée est validée par le processeur au moyen de résultats issus d'un questionnaire.

6. Processeur selon la revendication 5, dans lequel le questionnaire comprend des questions de dépistage et comprend en outre des questions déterminées à partir de la réponse de l'individu aux questions de dépistage.

7. Processeur selon la revendication 1, comprenant l'étape consistant à déterminer les informations de modification.

8. Processeur selon la revendication 1, comprenant l'étape durant laquelle l'individu effectue une tâche en ligne.

9. Processeur selon la revendication 1, comprenant l'étape consistant à surveiller des formes d'utilisation d'une machine par l'individu.

10. Processeur selon la revendication 1, comprenant l'étape consistant à surveiller électroniquement le comportement de l'individu.

11. Processeur selon la revendication 1, dans lequel la pluralité de valeurs est pondérée.

12. Processeur selon la revendication 1, dans lequel l'individu est un animal, y compris un être humain.
